# EUROPEAN PATENT SPECIFICATION

(11) **EP 2 572 690 B1**
(45) Date of publication and mention of the grant of the patent: **14.05.2014**
(21) Application number: 12185063.0
(22) Date of filing: 19.09.2012
(51) Int. Cl.: A61F 13/534, A61F 13/537

(54) **Resilient absorbent composite material**
Elastisches aufnahmefähiges Verbundstoffmaterial
Matériau composite absorbant élastique

(30) Priority: 20.09.2011 US 201113236777
(43) Date of publication of application: 27.03.2013
(73) Proprietor: McNEIL-PPC, Inc., Skillman, NJ 08558 (US)
(72) Inventor: Yang, Morris, Princeton Junction, New Jersey 08550 (US)
(74) Representative: Kirsch, Susan Edith

(56) References cited:
- WO-A1-01/26593
- US-B1- 6 403 857

## Description

### FIELD OF INVENTION

The present invention generally relates to an absorbent composite material and an absorbent article including such absorbent composite material. More particularly, the present invention relates to an absorbent composite material for use in sanitary absorbent articles such as sanitary napkins, pantiliners, diapers, adult incontinence products, and the like.

### BACKGROUND OF THE INVENTION

Lofty, resilient, non-woven webs, that is nonwoven webs that have a high degree of loft and the tendency to retain such loft in both the dry and wet state, are well known. In addition, it is well known that such lofty, resilient, nonwoven webs may be used in disposable sanitary products such as sanitary napkins, pantiliners, tampons, diapers, adult incontinence products, and the like. A perceived benefit of such lofty, resilient, nonwoven webs is that such materials may deliver enhanced comfort to a user of such disposable sanitary products since the lofty, resilient, nonwoven webs may tend to conform to, and move with, the user's body during use.

A problem with lofty, resilient, nonwoven webs is that due to the loft of such materials (i.e. their low density) such webs are not particularly absorbent. In addition, such materials may exhibit poor rewet properties. That is, such materials may release or "wet back" fluid when subjected to an external pressure.

In view of the foregoing there is a need for an absorbent composite material that includes an exceptionally soft, cushiony, nonwoven web and simultaneously provides superior fluid handling characteristics.

### SUMMARY OF THE INVENTION

In view of the foregoing, the present invention provides an absorbent composite material including a fibrous material including a plurality of individual fibers forming a fiber matrix and a binder material present in an amount from about 20% by to about 60% by weight of the fibrous material, the fibrous material having first and second opposed surfaces, superabsorbent polymer dispersed within the fiber matrix, an absorbent mixture arranged adjacent to the first surface of the fibrous material, the absorbent mixture including superabsorbent polymer and adhesive.

### BRIEF DESCRIPTION OF THE DRAWINGS

Examples of embodiments of the present invention will now be described with reference to the drawings, in which:
Fig. 1 is a sectional schematic view of an absorbent composite material according to the present invention;
Fig. 2 is a schematic view of an apparatus for making the absorbent composite material shown in Fig. 1;
Fig. 3 is a sectional schematic view of an absorbent composite material according to the present invention
Fig. 4 is a schematic view of an apparatus for making the absorbent composite material shown in Fig. 3;
Fig. 5 is a schematic view of the encircled portion of the apparatus shown in Fig. 4 depicting a needle employed in the apparatus, a top surface of a fibrous material, and absorbent fibers arranged on the top surface of the fibrous material;
Fig. 6 is a detailed view of the encircled portion of the needle shown in Fig. 5;
Figs. 7-10 depict the manner in which the needle impregnates the absorbent fiber within the fibrous material;
Fig. 11 is a perspective view of an absorbent article;
Fig. 12 is an exploded perspective view of the article shown in Fig. 11 revealing the constituent layers thereof;
Fig. 13 is sectional view taken along line x-x in Fig. 11 showing one embodiment of the absorbent article shown in Fig 11; and
Fig. 14 is sectional view taken along line x-x in Fig. 11 showing an alternate embodiment of the absorbent article shown in Fig. 11.

### DETAILED DESCRIPTION OF THE INVENTION

The present invention generally relates to an absorbent composite material for use in disposable absorbent articles such as sanitary napkins, pantiliners, absorbent products for incontinence, and other disposable sanitary absorbent articles worn close to a wearer's body. Although the absorbent composite material according to the present invention will be described herein with reference to a sanitary napkin, the absorbent composite material may be utilized in other disposable sanitary absorbent articles.

The absorbent composite material according to the present invention, as described in detail below, is structured and arranged such that it provides superior fluid absorbing properties and at the same time is "resilient". The term "resilient" as used herein means that the absorbent composite material tends to retain its shape both in the dry and wet states and when subjected to a compression force tends to recover its original shape when such force is removed. Absorbent articles according to the present invention including the inventive absorbent composite material are thin, flexible, resilient in the x, y and z directions, and exhibit superior fluid handling characteristics. "Resilient in the x, y and z direction" as used herein means the absorbent article exhibits resiliency properties in the transverse direction of the article, in the longitudinal direction of the article, and the direction extending into the article.

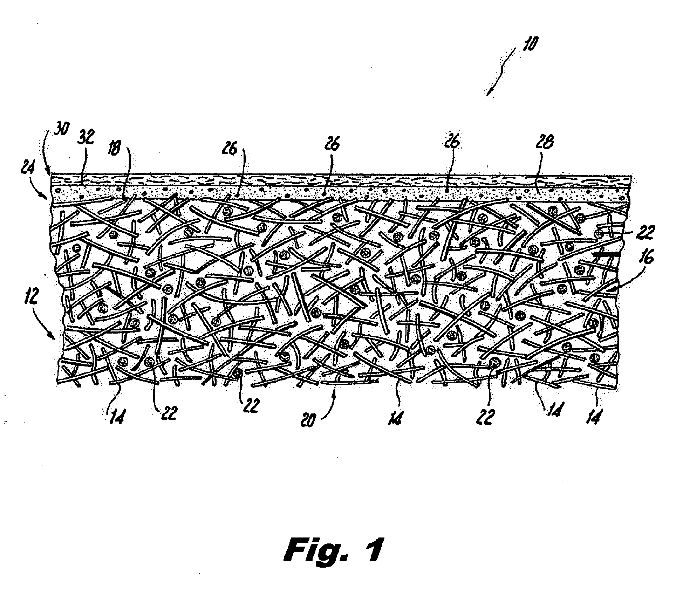

Reference is made to Fig. 1 which illustrates a sectional view of an absorbent composite material 10 according to the present invention. As shown, the absorbent composite material 10 includes, in part, a fibrous material 12 including a plurality of individual fibers 14 that form a fiber matrix 16. The fibrous material 12 generally includes a top (or first) surface 18 and an opposed bottom (or second) surface 20.

The fibrous material 12 is preferably a fibrous nonwoven material made by a known nonwoven manufacturing technique such as an airlaid process, a card and bind process or a resin and adhesive bond process. Preferably the nonwoven material is a "high loft" nonwoven. Specifically, the nonwoven preferably has a density lower than 0.05 g/cc, and preferably between about 0.01 g/cc and 0.03 g/cc. The individual fibers 14 forming the fibrous nonwoven material may be selected from fibers including synthetic, nonabsorbent fibers that may or may not be wettable. Specific fiber types include, but are not limited to, polyester, nylon, co-polyester, polyethylene, polypropylene, polylactic acid, and bicomponent fibers including these materials. Of course the fibrous nonwoven material may be formed from a single nonabsorbent fiber type listed above or alternatively may be formed from a mixture of the fiber types listed above.

The surface of the nonabsorbent fibers 14 forming the fibrous material 12 may be rendered wettable by treating such fibers with a suitable surface treatment, such as a surfactant or like. The fibrous material 12 preferably further includes a binder material, such as a latex binder. The binder material is preferably present in the fibrous material 12 in an amount that corresponds to between about 20% by weight to about 60% by weight of the fibrous material 12. The individual fibers 14 forming the fibrous material preferably have a denier in the range from about 5 to about 25, preferably from about 6 to about 10. Each of the fibers 14 forming the fibrous material preferably has a fiber diameter within the range of 11 µm and 100 µm. In preferred embodiments of the invention, the fibrous material 12 is completely free of cellulosic material.

The fibrous material 12 preferably has a basis weight in the range of about 50 gsm (g/m²) to about 150 gsm, preferably from about 60 gsm to about 90 gsm (including the binder material). The fibrous material 12 preferably has a thickness of between about 2 mm to about 6 mm as measured by a Ames Micrometer (Ames Waltman Mass., Model ADP1132, 175 g on the 1 1/8" foot = 0.384 psi). A fibrous material 12 particularly suitable for use in the present invention is a material made from a randomized web sprayed with binder from both sides of the web, having a basis weight of 86 gsm, formed from 100% 6 denier polyester fibers, having a thickness of about 3 mm, and including about 40% latex binder by weight, commercially available under product code SCN09-038 from Kem-Wove, Inc., Charlotte, NC.

As shown in Fig. 1, the absorbent composite material 10 further includes superabsorbent polymer 22 dispersed within the fiber matrix 16. The superabsorbent polymer 22 is preferably present in the fibrous material 12 in an amount that corresponds to between about 100% to about 150% by weight of the fibrous material 12, e.g. between about 80 gsm and about 120 gsm.

For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the products offered by Sumitomo Seika Chemicals Co., Ltd. of Osaka, Japan, under the designation of SA70 and BA40B. The superabsorbent polymer 22 present in the absorbent composite 10 may consist of a single superabsorbent such as SA70 or alternatively the superabsorbent polymer 22 may consist of a mixture of superabsorbent polymers such as a mixture of SA70 and BA40B. In addition, different superabsorbent polymers may be arranged in a layered arrangement, for example the faster rate BA40B may be arranged above the SA70.

Referring again to Fig. 1, the absorbent composite material 10 further includes an absorbent mixture 24 arranged adjacent to the top surface 18 of the fibrous material 12. The absorbent mixture 24 includes superabsorbent polymer 26 and adhesive 28. Preferably the absorbent mixture 24 consists of a mixture of superabsorbent polymer 26 and adhesive 28. The mixture 24 preferably includes between about 90% and about 98% superabsorbent by weight and between 10% and about 2% adhesive by weight. Preferred superabsorbents for use in the mixture include Sumitomo SA70 and Sumitomo BA40B, commercially available from Sumitomo Seika Chemicals, Co., Ltd., Osaka, Japan. Preferably a hot melt adhesive is used as the adhesive in the mixture 24. A particularly suitable adhesive is HB Fuller NW 1023 hot melt adhesive, commercially available from HB Fuller Company, St. Paul, MN. The mixture 24 is preferably applied to the top surface 18 of the fibrous material 12 in an add on amount of between about 80 gsm and 100 gsm. This add on amount of the mixture 24 corresponds to between about 100% to 150% by weight of the fibrous material 12.

In lieu of the absorbent mixture 24 described above, a simple layer of adhesive could alternatively be applied adjacent to the top surface 18 of the fibrous material 12. In such an embodiment the adhesive would not provide additional absorbency to the composite material 10 but would rather simply help prevent the superabsorbent polymer 22 from "dusting out" of the fiber matrix 16.

Referring again to Fig. 1, the absorbent composite material 10 further includes a superabsorbent retention layer 30. As shown in Fig. 1, the superabsorbent retention layer 30 is arranged adjacent to a top surface 32 of the mixture 24 and is held in place by the mixture 24. The superabsorbent retention layer 30 functions to prevent the superabsorbent polymer 22 from "dusting out" of the fiber matrix 16.

The superabsorbent retention layer 30 may also be adapted to wick fluid in a longitudinal and transverse direction of the composite material 10 so that the composite material 10 can fully utilize its absorbent attributes.

The superabsorbent retention layer 30 may comprise or consist of a wetlaid tissue having a basis weight in the range of about 10 gsm to about 20 gsm, such as a 17 gsm wetlaid tissue commercially available as Little Rapids type 2004 wetlaid tissue, Little Rapids Corp., Green Bay, Wisconsin.

The superabsorbent retention layer 30 may comprises or consist of fibrous material including wood pulp fibers, polyester fibers, rayon fibers, or combinations thereof. The superabsorbent retention layer 30 may also comprise thermoplastic fibers for the purpose of stabilizing the layer and maintaining its structural integrity. Examples of materials suitable for the superabsorbent retention layer 30 are through air bonded pulp sold by Buckeye Technologies, Inc. of Memphis, Tenn. under the designation Vizorb 3008 which has a basis weight of 100 gsm and Vizorb 3010 which has a basis weight of 90 gsm.

Another example of a material suitable for use as the superabsorbent retention layer 30 is a calendared airlaid material of the type commercially available from EAM Corporation, Jessup, GA under the designation Novathin. When calendared airlaid materials are used as the superabsorbent retention layer 30 such materials preferably have a basis weight in the range of about 40 gsm to about 90 gsm. The superabsorbent retention layer 30 is preferably free of superabsorbent polymer.

Another example of a materials suitable for use as the superabsorbent retention layer 30 are commercially available paper towel materials.

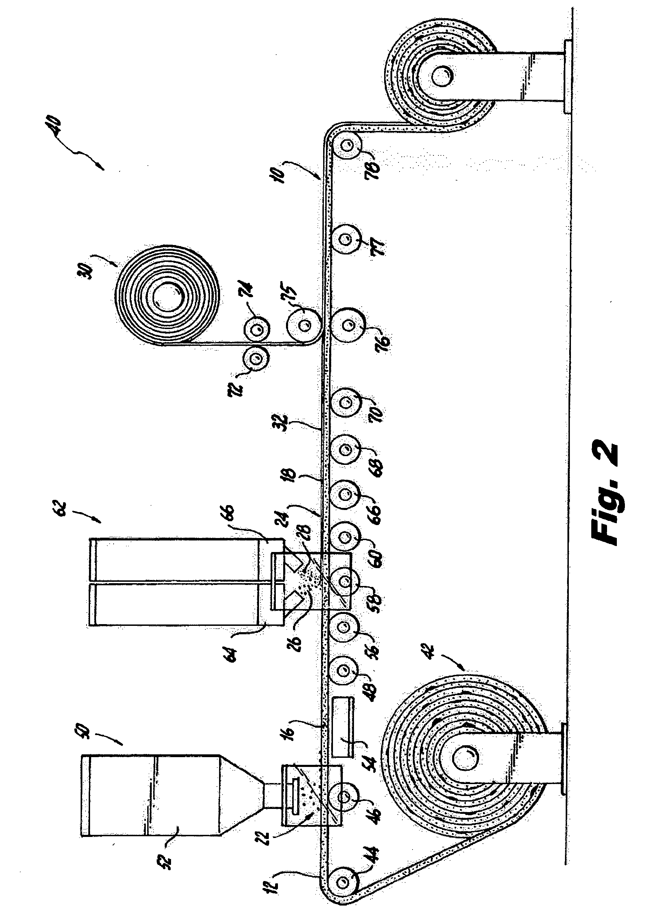

A method of making the absorbent composite material 10 will now be described with reference to Fig. 2 which depicts a schematic representation of an apparatus 40 for making the absorbent composite material 10. As shown in Fig. 2, a web of fibrous material 12 is fed from a supply roll 42 and conveyed in a machine direction by a plurality of rolls 44, 46 and 48 to a superabsorbent application station 50. The superabsorbent application station 50 comprises a metering device 52 structured and arranged to apply a selected amount of superabsorbent polymer 22 to a top surface 18 of the fibrous material 12. Metering devices for applying particulate material to a substrate are well known to those of skill in the art. Of course, any suitable means known to those of skill in the art, such as a pressure fed nozzle, a brush metering roll, or the like, may be used to apply the superabsorbent polymer material 22 to the top surface 18 of the fibrous material 12. After the superabsorbent polymer material 22 is applied to the top surface 18 of the fibrous material 12, the fibrous material 12 is conveyed over a high frequency vibrator 54 that functions to mechanically force the superabsorbent polymer 22 into the fibrous matrix 16 of the fibrous material 12. Alternatively, a vacuum arranged below the bottom surface 20 of the fibrous material 12 may be used to draw the superabsorbent polymer 22 into the fibrous matrix 16.

Thereafter, the fibrous material 12 is further conveyed in a machine direction by a plurality of rolls 56, 58 and 60 to an absorbent mixture application station 62. The absorbent mixture application station 62 includes a metering device 64 for applying a stream of superabsorbent polymer 26 to the top surface 18 of the fibrous material 12. The absorbent mixture application station 62 further includes a hot melt adhesive applicator 66 which is directed at the fibrous material 12 and adapted to apply a stream of adhesive 28 to the top surface 18 of the fibrous material 12. Preferably the adhesive 28 stream and superabsorbent polymer 26 stream mix in mid air, thereby forming the absorbent mixture 24, and then the absorbent mixture 24 is deposited on the top surface 18 of the fibrous material 12.

Thereafter, the fibrous material 12 is further conveyed in a machine direction by a plurality of rolls 66, 68 and 70. The superabsorbent retention layer 30 is then arranged adjacent to the top surface 32 of the mixture 24 by means of rolls 72 and 74. Then the substrate 12 and superabsorbent retention layer 30 are passed through nip rolls 75 and 76 to thereby adhere the superabsorbent retention layer 30 to the absorbent mixture 24. Thereafter the completed absorbent composite material 10 is further conveyed in a machine direction by rolls 77 and 78 and may be arranged in a rolled form for storage or may be further conveyed for incorporation into a disposable sanitary absorbent product such as a sanitary napkin, pantiliner, tampon, diaper, adult incontinence product, or the like.

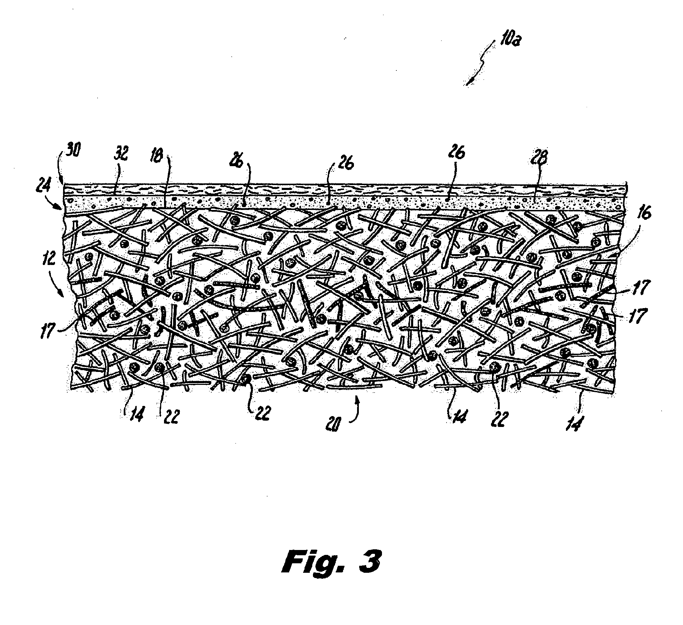

Reference is made to Fig. 3 which illustrates a sectional view of an absorbent composite material 10a according to the present invention. Absorbent composite material 10a includes the same features as described above with regard to composite material 10, however absorbent composite material 10a further includes a plurality of absorbent fibers 17. As shown, the absorbent fibers 17 are impregnated within the fiber matrix 16. The absorbent fibers 17 are preferably present in the fibrous material 12 in an amount between 5% to about 100% by weight of the fibrous material 12, corresponding to about 3 gsm to about 60 gsm. The absorbent fibers 17 are preferably selected from cellulosic fiber types, such as, but not limited to, hard wood pulp, soft wood pulp, rayon, and cotton. The absorbent material 10 may include a single absorbent fiber type of those listed above or in the alternative may include multiple fiber types of those listed above (i.e. a mixture of absorbent fibers). Each of the absorbent fibers 17 preferably has fiber diameter within the range of 10 µm and 40 µm. The individual fibers 14 forming the fibrous material 12 and the absorbent fibers 17 are selected such that each of the individual fibers 14 has a fiber diameter that is at least 1 µm greater than a fiber diameter of each of the absorbent fibers 17.

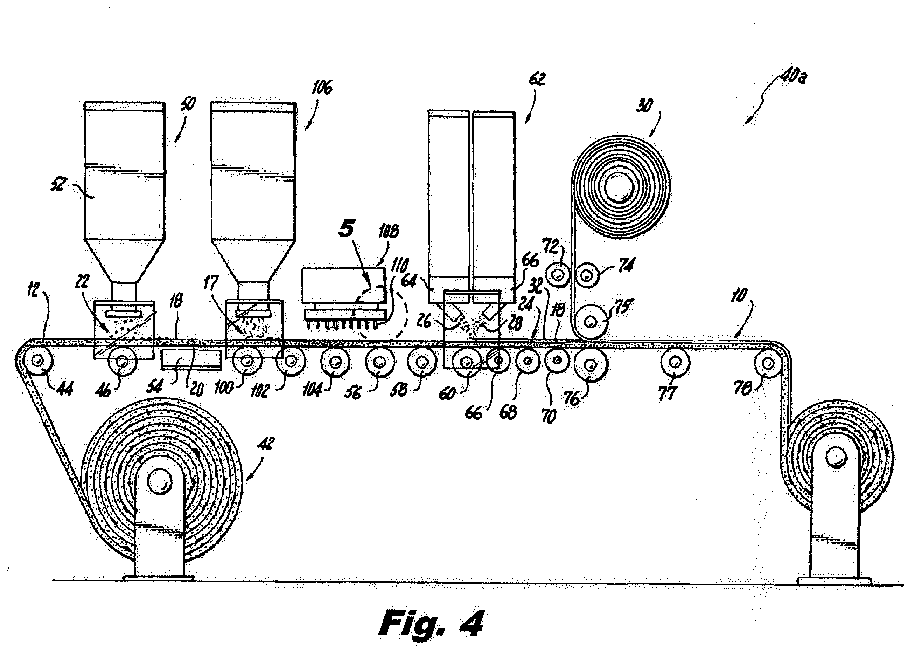

A method of making the absorbent composite material 10a will now be described with reference to Fig. 4 which depicts a schematic representation of an apparatus 40a for making the absorbent composite material 10. As shown in Fig. 4, a web of fibrous material 12 is fed from a supply roll 42 and conveyed in a machine direction by a plurality of rolls 44, 46 and 48 to a superabsorbent application station 50. The superabsorbent application station 50 comprises a metering device 52 structured and arranged to apply a selected amount of superabsorbent polymer 22 to a top surface 18 of the fibrous material 12. Metering devices for applying particulate material to a substrate are well known to those of skill in the art. Of course, any suitable means known to those of skill in the art, such as a pressure fed nozzle, a brush metering roll, or the like, may be used to apply the superabsorbent polymer material 22 to the top surface 18 of the fibrous material 12. After the superabsorbent polymer material 22 is applied to the top surface 18 of the fibrous material 12, the fibrous material 12 is conveyed over a high frequency vibrator 54 that functions to mechanically force the superabsorbent polymer 22 into the fibrous matrix 16 of the fibrous material 12. Alternatively, a vacuum arranged below the bottom surface 20 of the fibrous material 12 may be used to draw the superabsorbent polymer 22 into the fibrous matrix 16.

Thereafter the fibrous material is further conveyed in a machine direction by rolls 100, 102 and 104 to a gravity fed hopper 106, or the like, that is utilized to apply a selected amount of absorbent fibers 17 to a top surface 18 of the fibrous material 12. If required, the material forming the absorbent fibers may be fed through a lickerin or a hammermill prior to the absorbent fibers being fed into the gravity fed hopper (not shown in the drawings). Thereafter, the fibrous material 12 is further conveyed in a machine direction and passed through a conventional needlepunch apparatus 108 of the type known to those of skill in the art. The needlepunch apparatus 108 functions to impregnate the absorbent fibers 17 within the fibrous material 12 by means of a plurality of needles 110.

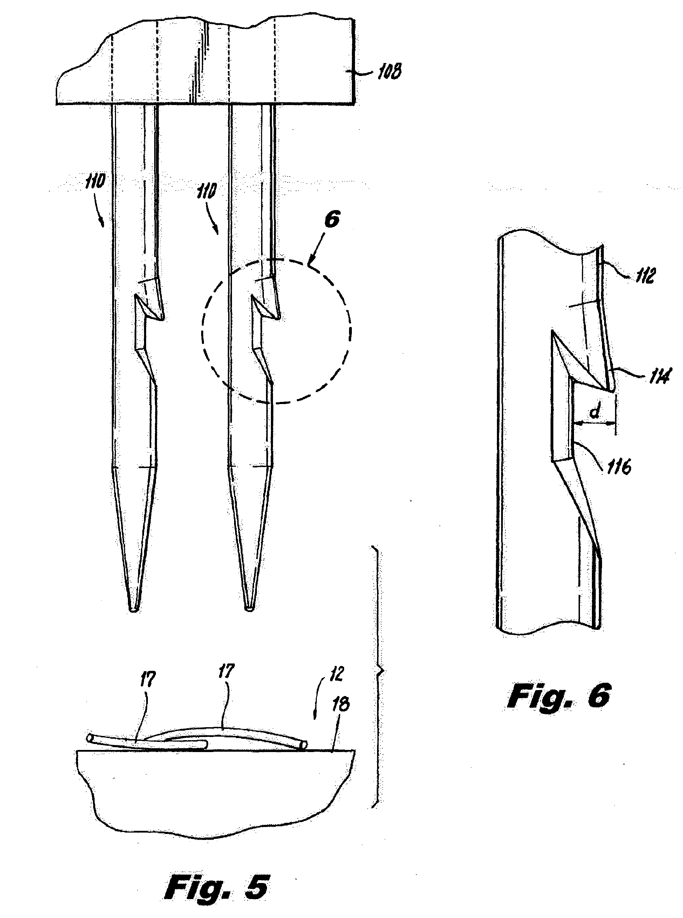

As is known to those of skill in the art, a conventional needlepunch apparatus includes a plurality of needles that are normally adapted to mechanically orient and interlock the fibers of a spunbonded or carded web. In the present invention, the needles 110 of the needlepunch apparatus 108 are used to impregnate absorbent fibers 17 within the fibrous material 12. A needle 110 suitable for use in the method described herein is depicted in Fig. 5 and Fig. 6. As shown in Fig. 6, the needle 110 generally includes a blade 112, a barb 114, and a throat section 116. The total barb depth of the barb 114 is indicated by letter "d" in the Fig. 6.

For purposes of the present invention, it is critical that the barb depth "d" is selected such that a radius of each of the absorbent fibers 17 is smaller than the barb depth "d". The radius of each absorbent fiber 17 is at least 0.5 µm smaller, for example 1 µm smaller than the barb depth. In addition the barb depth "d" should be selected such that each of the individual fibers 14 of the fibrous material 12 has a radius that is larger than the barb depth "d". The radius of each individual fiber 14 of the fibrous material 12 is at least 0.5 µm larger, for example 1 µm larger than the barb depth. If you have a multiple denier fibrous material 12, the diameter of the smallest diameter fiber 14 must be larger than the diameter of each of the absorbent fibers 17.

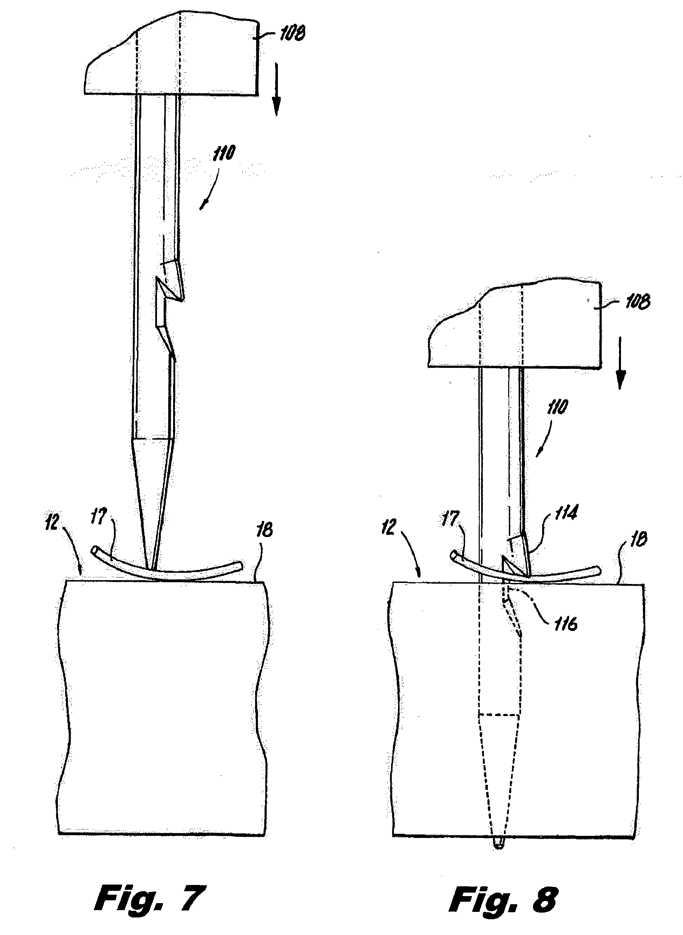

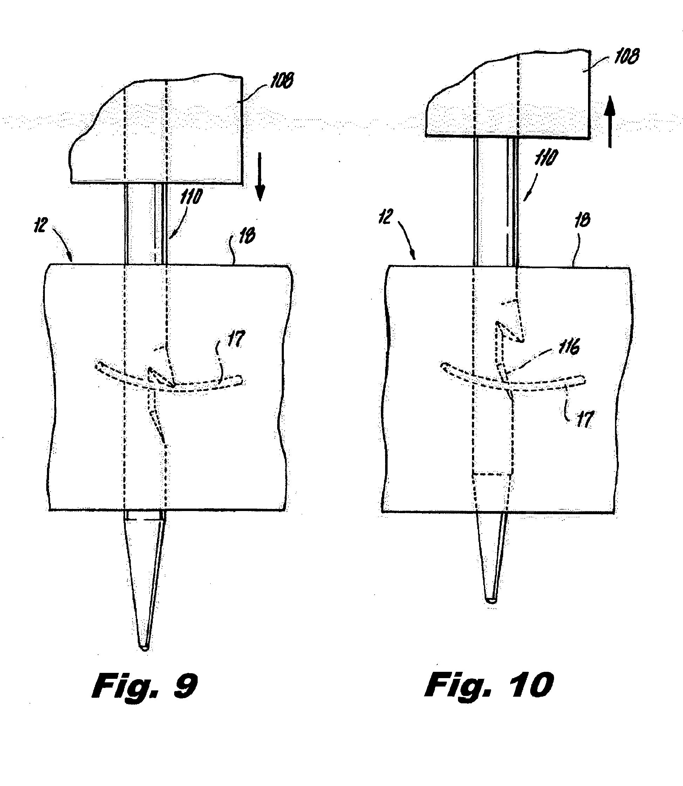

By selecting barb depth "d" as described above, the plurality of needles 110 in the needlepunch apparatus effectively grasp the absorbent fibers 17 and thus can impregnate such absorbent fibers 17 within the fibrous material 12, as shown in Figs 7-10. On the other hand, the plurality of needles 110 will not grasp the individual fibers 14 of the fibrous material 12 and thus will not destroy the "high loft" properties of the fibrous material 12. In this manner the final absorbent composite material 10 is provided with superior fluid handling properties while still retaining the high loft properties of the fibrous material 12. Needles particularly useful in the present method are commercially available from the Foster Needle Co., Inc., Manatowoc, Wisconsin, under product designation "The Foster Formed Barb".

Thereafter, the fibrous material 12 is further conveyed in a machine direction by a plurality of rolls 56, 58 and 60 to an absorbent mixture application station 62. The absorbent mixture application station 62 includes a metering device 64 for applying a stream of superabsorbent polymer 26 to the top surface 18 of the fibrous material 12. The absorbent mixture application station 62 further includes a hot melt adhesive applicator 66 which is directed at the fibrous material 12 and adapted to apply a stream of adhesive 28 to the top surface 18 of the fibrous material 12. Preferably the adhesive 28 stream and superabsorbent polymer 26 stream mix in mid air, thereby forming the absorbent mixture 24, and then the absorbent mixture 24 is deposited on the top surface 18 of the fibrous material 12.

Referring again to Fig. 4, the fibrous material 12 is further conveyed in a machine direction by a plurality of rolls 66, 68 and 70. The superabsorbent retention layer 30 is then arranged adjacent to the top surface 32 of the mixture 24 by means of rolls 72 and 74. Then the substrate 12 and superabsorbent retention layer 30 are passed through nip rolls 75 and 76 to thereby adhere the superabsorbent retention layer 30 to the absorbent mixture 24. Thereafter the completed absorbent composite material 10 is further conveyed in a machine direction by rolls 77 and 78 and may be arranged in a rolled form for storage or may be further conveyed for incorporation into a disposable sanitary absorbent product such as a sanitary napkin, pantiliner, tampon, diaper, adult incontinence product, or the like.

Absorbent composite materials 10 according to the to the present invention, as described herein above, are thin, lofty and exhibit superior resiliency properties in both the dry and wet state. Surprisingly, absorbent composite materials 10 according to the present invention exhibit the above properties while at the same time exhibiting superior fluid handling characteristics.

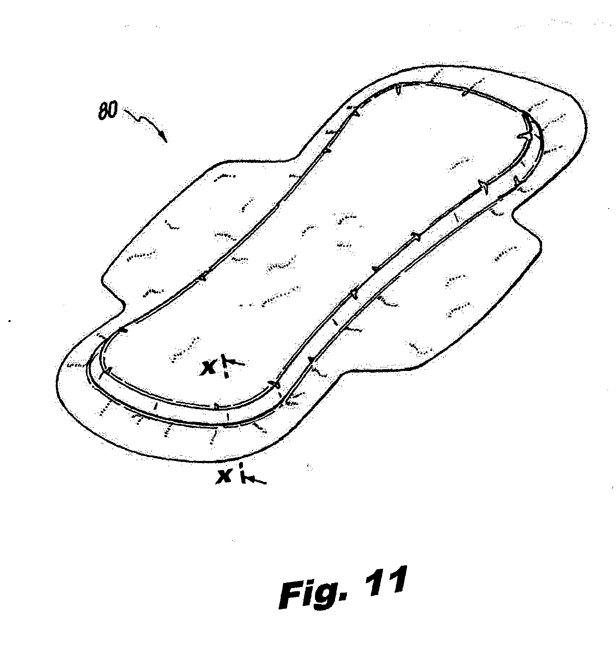

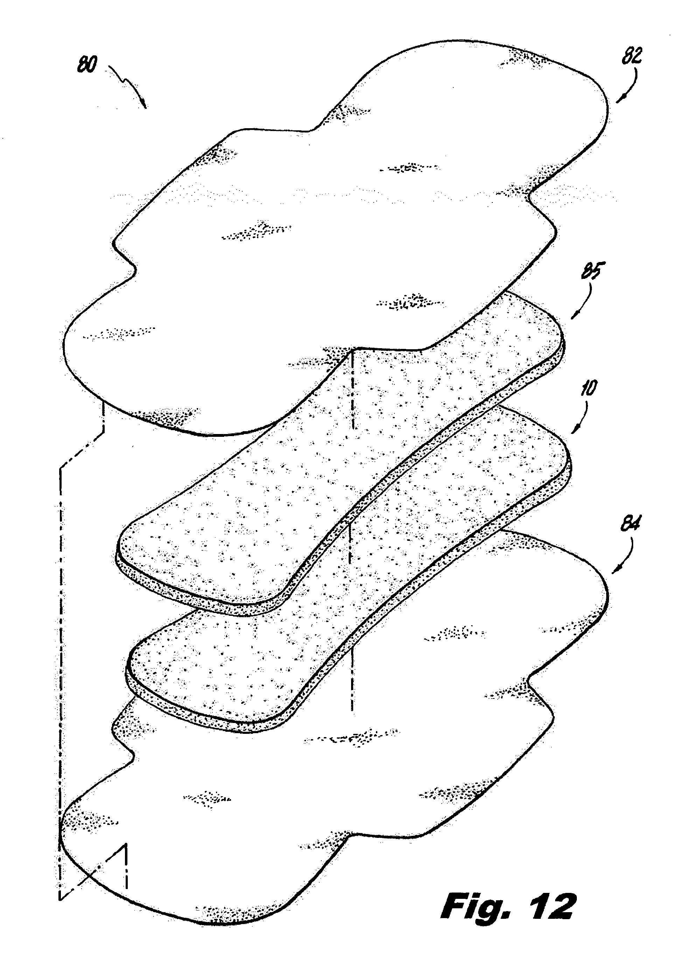

Reference is made to Figs. 11-14 which depict an absorbent article, and in particular a sanitary napkin 80. As shown in Fig. 12, the sanitary napkin 80 generally includes a liquid permeable cover layer 82, a liquid impermeable barrier layer 84 and the absorbent composite material 10 arranged therebetween. The sanitary napkin 80 may further optionally include a fluid distribution layer 85 arranged between the cover layer 82 and the absorbent composite material 10.

The cover layer 82 may be a relatively low density, bulky, high-loft non-woven web material. The cover layer 82 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 82 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer and/or to the barrier layer.

The cover layer 82 preferably has a relatively high degree of wettability, although the individual fibers comprising the cover may not be particularly hydrophilic. The cover material should also contain a great number of relatively large pores. This is because the cover layer 82 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 82 should not lose their physical properties when they are wetted, in other words they should not collapse or lose their resiliency when subjected to water or body fluid. The cover layer 82 may be treated to allow fluid to pass through it readily. The cover layer 82 also functions to transfer the fluid quickly to the underlying layers of the napkin. Thus, the cover layer 82 is advantageously wettable, hydrophilic and porous. When composed of synthetic hydrophobic fibers such as polyester or bi-component fibers, the cover layer 82 may be treated with a surfactant to impart the desired degree of wettability.

Nonwoven cover materials particularly suitable for use in the present invention are hot-through air bonded cover materials commercially available from Shalag Industries, Ltd., Upper Galilee, Israel, under product codes STA4ETW27, STA5ETW27, STAFEPW27, STA5EPW27, STAPPER22 and STAFETW22.

Alternatively, the cover layer 82 can also be made of polymer film having large pores. Because of such high porosity, the film accomplishes the function of quickly transferring body fluid to the inner layers of the underlying absorbent layers. The cover layer 82 may be attached to the underlying absorbent composite material 10 and/or the barrier layer 84, by adhesion and/or other suitable means know to those of skill in the art. The cover layer 82 may also be attached to the underlying fluid distribution layer 85 if such layer is employed.

Underlying the cover layer 82 is the optional fluid distribution layer 85. The fluid distribution layer 85 functions to receive body fluid from the cover layer 82 and hold the same until the absorbent composite 10 has an opportunity to absorb the fluid. The fluid distribution layer 85 is preferably more dense than and has a larger proportion of smaller pores than the cover layer 82. These attributes allow the fluid distribution layer 85 to contain the body fluid and hold it away from the outer side of the cover layer 82, thereby preventing fluid from rewetting the cover layer 82.

The fluid distribution layer 85 may consist of fibrous material including wood pulp fibers, polyester fibers, rayon fibers, or combinations thereof. The fluid distribution layer may also comprise thermoplastic fibers for the purpose of stabilizing the layer and maintaining its structural integrity. Examples of materials suitable for the fluid distribution layer 85 are through air bonded pulp materials sold by Buckeye Technologies, Inc. of Memphis, Tenn. under the designation Vizorb 3008 which has a basis weight of 100 gsm and Vizorb 3010 which has a basis weight of 90 gsm.

Another example of a material suitable for use as the fluid distribution layer 85 is a calendared airlaid material of the type commercially available from EAM Corporation, Jessup, GA under the designation Novathin. The fluid distribution layer 85 is preferably free of superabsorbent polymer.

Underlying absorbent composite material 10 is a barrier layer 84 comprising a liquid-impervious film material so as to prevent liquid that is entrapped in the absorbent composite layer 10 from egressing the sanitary napkin and staining the wearer's undergarment. The barrier layer 84 is preferably made of polymeric film, although it may be made of liquid impervious, air-permeable material such as repellent-treated non-woven or micropore films or foams.

The barrier layer 84 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 82 and the barrier layer 84 are preferably joined along their marginal portions so as to form an enclosure or flange seal that maintains the absorbent composite layer 10 captive. The joint may be made by means of adhesives, heat-bonding, ultrasonic bonding, radio frequency sealing, mechanical crimping, and the like and combinations thereof.

Positioning adhesive may be applied to a garment facing surface of the barrier layer 84 for securing the napkin 80 to a garment during use. The positioning adhesive may be covered with removable release paper so that the positioning adhesive is covered by the removable release paper prior to use.

Absorbent articles may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

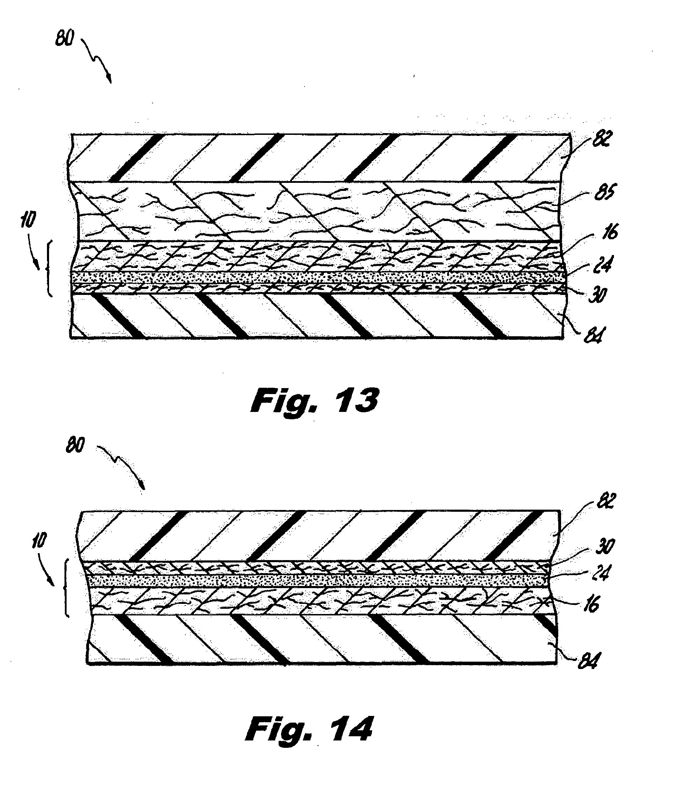

Reference is now made to Figs. 13 and 14 which depict sectional views taken along line x-x in Fig. 11 and depict alternative embodiments of the sanitary napkin 80 shown in Fig. 11. As shown in Fig. 13, the absorbent composite material 10 may be arranged in the sanitary napkin 80 such that the superabsorbent retention layer 30 is arranged in adjacent surface to surface contact with the barrier layer 84. The embodiment of the napkin 80 shown in Fig. 13 includes a fluid distribution layer 85. Of course, however the fluid distribution layer 85 could be omitted such that the second surface 20 of the fibrous material 12 is arranged in surface to surface contact with the cover layer 82.

Alternatively, as shown in Fig. 14, the absorbent composite material 10 may be arranged in the sanitary napkin 80 such that the superabsorbent retention layer 30 is arranged in adjacent surface to surface contact with the cover layer 82.

Absorbent articles as described above are thin, cushiony, soft, exhibit superior resiliency properties in both the dry and wet state, and also exhibit superior fluid handling characteristics.

## Claims

1. An absorbent composite material comprising:
a fibrous material including a plurality of individual fibers forming a fiber matrix and a binder material present in an amount from about 20% by to about 60% by weight of the fibrous material, the fibrous material having first and second opposed surfaces;
superabsorbent polymer dispersed within the fiber matrix;
an absorbent mixture arranged adjacent to the first surface of the fibrous material, the absorbent mixture comprising superabsorbent polymer and adhesive.

2. The absorbent composite material according to claim 1, wherein the fibrous material is a nonwoven material having a density lower than 0.05 g/cc, preferably wherein the fibrous material is a high loft nonwoven material having a density between 0.01 g/cc and 0.03 g/cc.

3. The absorbent composite material according to claim 1 or claim 2, wherein each of the individual fibers is nonabsorbent.

4. The absorbent composite material according to any preceding claim, wherein each of the individual fibers are selected from the group consisting of polyester, nylon, co-polyester, polyethylene, polypropylene, polylactic acid, and bicomponent fibers including these materials.

5. The absorbent composite material according to any preceding claim, wherein each of the individual fibers has a denier in the range of from about 5 to about 25, or in the range of from about 6 to about 10.

6. The absorbent composite material according to any preceding claim, wherein the fibrous material is completely free of cellulosic material.

7. The absorbent composite material according to any preceding claim, wherein the fibrous material has a basis weight of between about 50 gsm and 150 gsm.

8. The absorbent composite material according to any preceding claim, wherein the superabsorbent polymer dispersed within the fiber matrix is present in the fibrous material in an amount between about 50% to about 150 % by weight of the fibrous material.

9. The absorbent composite material according to any preceding claim, wherein the superabsorbent polymer dispersed within the fiber matrix is present in an amount of from about 80 gsm to about 150 gsm.

10. The absorbent composite material according to any preceding claim, wherein the absorbent mixture consists of superabsorbent polymer and adhesive.

11. The absorbent composite material according to any preceding claim, wherein the absorbent mixture includes between about 90% and about 98% superabsorbent by weight and about 10% and 2% adhesive by weight.

12. The absorbent composite material according to any preceding claim, further comprising a superabsorbent retention layer.

13. The absorbent composite material according to claim 12, wherein the superabsorbent retention layer is arranged adjacent to a top surface of the absorbent mixture.

14. The absorbent composite material according to claim 12 or claim 13, wherein the superabsorbent retention layer is a tissue material, or wherein the superabsorbent retention layer is a through air bonded pulp material, or wherein the superabsorbent retention layer is a calendared airlaid material.

15. The absorbent composite material according to any preceding claim, further comprising a plurality of absorbent fibers arranged within the fiber matrix.

16. The absorbent composite material according to claim 15, wherein the absorbent fibers are present in the fibrous material in an amount between about 5% and about 100% by weight of the fibrous material.

## Patentansprüche

1. Absorbierender Verbundstoff, umfassend:
ein Fasermaterial, mit mehreren, eine Fasermatrix bildenden Einzelfasern und einem in einer auf das Fasermaterial bezogenen Menge von etwa 20 bis etwa 60 Gew.-% vorliegenden Bindemittel, wobei das Fasermaterial über eine erste und eine zweite Oberfläche verfügt, wobei die beiden Oberflächen einander gegenüberliegen,
einen in der Fasermatrix verteilten Superabsorber und
eine an die erste Oberfläche des Fasermaterials angrenzend angeordnete absorbierende Mischung, wobei die absorbierende Mischung einen Superabsorber und einen Klebstoff umfasst.

2. Absorbierender Verbundstoff nach Anspruch 1, bei dem es sich bei dem Fasermaterial um einen Vliesstoff mit einer Dichte von weniger als 0,05 g/cm³ handelt, wobei es sich bei dem Fasermaterial bevorzugt um einen Volumenvliesstoff mit einer Dichte zwischen 0,01 g/cm³ und 0,03 g/cm³ handelt.

3. Absorbierender Verbundstoff nach Anspruch 1 oder 2, bei dem die Einzelfasern jeweils nichtabsorbierend sind.

4. Absorbierender Verbundstoff nach einem der vorhergehenden Ansprüche, bei dem die Einzelfasern jeweils ausgewählt sind aus der Gruppe bestehend aus Polyester, Polyamid, Copolyester, Polyethylen, Polypropylen, Polymilchsäure und auf diesen Stoffen basierenden Bikomponentenfasern.

5. Absorbierender Verbundstoff nach einem der vorhergehenden Ansprüche, bei dem die Einzelfasern über einen Einzeltiter von etwa 5 bis etwa 25 den oder von etwa 6 bis etwa 10 den verfügen.

6. Absorbierender Verbundstoff nach einem der vorhergehenden Ansprüche, bei dem das Fasermaterial überhaupt kein Cellulosematerial enthält.

7. Absorbierender Verbundstoff nach einem der vorhergehenden Ansprüche, bei dem das Fasermaterial über ein zwischen etwa 50 g/m² und 150 g/m² liegendes Flächengewicht verfügt.

8. Absorbierender Verbundstoff nach einem der vorhergehenden Ansprüche, bei dem das Fasermaterial den in der Fasermatrix verteilten Superabsorber in einer auf das Fasermaterial bezogenen Menge zwischen etwa 50 und etwa 150 Gew.-% enthält.

9. Absorbierender Verbundstoff nach einem der vorhergehenden Ansprüche, bei dem der in der Fasermatrix verteilte Superabsorber in einer Menge von etwa 80 g/m² bis etwa 150 g/m² vorliegt.

10. Absorbierender Verbundstoff nach einem der vorhergehenden Ansprüche, bei dem die absorbierende Mischung aus Superabsorber und Klebstoff besteht.

11. Absorbierender Verbundstoff nach einem der vorhergehenden Ansprüche, bei dem die absorbierende Mischung zwischen etwa 90 und etwa 98 Gew.-% Superabsorber und etwa 10 und 2 Gew.-% Klebstoff enthält.

12. Absorbierender Verbundstoff nach einem der vorhergehenden Ansprüche, ferner umfassend eine superabsorbierende Retentionsschicht.

13. Absorbierender Verbundstoff nach Anspruch 12, bei dem die superabsorbierende Retentionsschicht an eine Oberseite der absorbierenden Mischung angrenzend angeordnet ist.

14. Absorbierender Verbundstoff nach Anspruch 12 oder 13, bei dem es sich bei der superabsorbierenden Retentionsschicht um ein Tissue-Material oder um einen durchluftverfestigten Zellstoff oder um ein kalendriertes Trockenvlies handelt.

15. Absorbierender Verbundstoff nach einem der vorhergehenden Ansprüche, ferner umfassend mehrere in der Fasermatrix angeordnete absorbierende Fasern.

16. Absorbierender Verbundstoff nach Anspruch 15, bei dem das Fasermaterial die absorbierende Faser in einer auf das Fasermaterial bezogenen Menge von zwischen etwa 5 Gew.-% und etwa 100 Gew.-% enthält.

## Revendications

1. Matériau composite absorbant comprenant :
un matériau fibreux comportant une pluralité de fibres individuelles formant une matrice de fibres et un matériau liant présent dans une quantité d'environ 20 % à environ 60 % en poids du matériau fibreux, le matériau fibreux ayant une première et une deuxième surface opposées ;
un polymère superabsorbant dispersé à l'intérieur de la matrice de fibres ;
un mélange absorbant disposé au voisinage de la première surface du matériau fibreux, le mélange absorbant comprenant un polymère superabsorbant et un adhésif.

2. Matériau composite absorbant selon la revendication 1, dans lequel le matériau fibreux est une matière non tissée ayant une masse volumique inférieure à 0,05 g/cm³, de préférence dans lequel le matériau fibreux est une matière non tissée à pouvoir gonflant élevé ayant une masse volumique comprise entre 0,01 g/cm³ et 0,03 g/cm³.

3. Matériau composite absorbant selon la revendication 1 ou la revendication 2, dans lequel chacune des fibres individuelles est non absorbante.

4. Matériau composite absorbant selon l'une quelconque des revendications précédentes, dans lequel chacune des fibres individuelles est choisie dans le groupe constitué par le polyester, le nylon, le copolyester, le polyéthylène, le polypropylène, l'acide polylactique, et les fibres bicomposants comportant ces matières.

5. Matériau composite absorbant selon l'une quelconque des revendications précédentes, dans lequel chacune des fibres individuelles a un denier dans la gamme d'environ 5 à environ 25, ou dans la gamme d'environ 6 à environ 10.

6. Matériau composite absorbant selon l'une quelconque des revendications précédentes, dans lequel le matériau fibreux est totalement dépourvu de matière cellulosique.

7. Matériau composite absorbant selon l'une quelconque des revendications précédentes, dans lequel le matériau fibreux a une masse surfacique comprise entre environ 50 g/m² et 150 g/m².

8. Matériau composite absorbant selon l'une quelconque des revendications précédentes, dans lequel le polymère superabsorbant dispersé à l'intérieur de la matrice de fibres est présent dans le matériau fibreux dans une quantité comprise entre environ 50 % et environ 150 % en poids du matériau fibreux.

9. Matériau composite absorbant selon l'une quelconque des revendications précédentes, dans lequel le polymère superabsorbant dispersé à l'intérieur de la matrice de fibres est présent dans une quantité d'environ 80 g/m² à environ 150 g/m².

10. Matériau composite absorbant selon l'une quelconque des revendications précédentes, dans lequel le mélange absorbant est composé d'un polymère superabsorbant et d'un adhésif.

11. Matériau composite absorbant selon l'une quelconque des revendications précédentes, dans lequel le mélange absorbant comporte entre environ 90 % et environ 98 % de superabsorbant en poids et entre environ 10 % et 2 % d'adhésif en poids.

12. Matériau composite absorbant selon l'une quelconque des revendications précédentes, comprenant en outre une couche de rétention superabsorbante.

13. Matériau composite absorbant selon la revendication 12, dans lequel la couche de rétention superabsorbante est disposée au voisinage d'une surface supérieure du mélange absorbant.

14. Matériau composite absorbant selon la revendication 12 ou la revendication 13, dans lequel la couche de rétention superabsorbante est une matière tissée, ou dans lequel la couche de rétention superabsorbante est une matière pâteuse liée par soufflage transversal d'air, ou dans lequel la couche de rétention superabsorbante est une matière air-laid calandrée.

15. Matériau composite absorbant selon l'une quelconque des revendications précédentes, comprenant en outre une pluralité de fibres absorbantes disposées à l'intérieur de la matrice de fibres.

16. Matériau composite absorbant selon la revendication 15, dans lequel les fibres absorbantes sont présentes dans le matériau fibreux dans une quantité comprise entre environ 5 % et environ 100 % en poids du matériau fibreux.
